# EUROPEAN PATENT SPECIFICATION

(11) **EP 4 087 521 B1**
(45) Date of publication and mention of the grant of the patent: **29.10.2025**
(21) Application number: 21710668.1
(22) Date of filing: 10.02.2021
(51) Int. Cl.: A61F 2/24

(54) **PROSTHETIC HEART VALVE**
HERZKLAPPENPROTHESE
VALVULE PROTHÉTIQUE

(30) Priority: 19.02.2020 US 202062978455 P
(43) Date of publication of application: 16.11.2022
(73) Proprietor: Edwards Lifesciences Corporation, Irvine, CA 92614-5688 (US)
(72) Inventor: YOHANAN, Ziv, 30889 Caesarea (IL); NIR, Noam, 30889 Caesarea (IL); SHERMAN, Elena, 30889 Caesarea (IL)
(74) Representative: Venner, Julia Ann
(86) International application number: PCT/US2021/017336
(87) International publication number: WO 2021/167819

(56) References cited:
- WO-A2-2012/048035
- US-A1- 2009 157 175
- US-A1- 2017 231 761
- US-A1- 2018 325 665

## Description

### CROSS-REFERENCED TO RELATED APPLICATION

The present application claims the benefit of U.S. Provisional Application No. 62/978,455, filed February 19, 2020.

### FIELD

The present disclosure relates to prosthetic heart valves and to methods for assembling prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery device and advanced through the patient's vasculature (*e.g*., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery device so that the prosthetic valve can self-expand to its functional size.

Prosthetic valves that rely on a mechanical actuator for expansion can be referred to as "mechanically expandable" prosthetic heart valves. The actuator typically takes the form of pull cables, sutures, wires and/or shafts that are configured to transmit expansion forces from a handle of the delivery apparatus to the prosthetic valve.

Most expandable, transcatheter heart valves comprise a cylindrical metal frame or stent and prosthetic leaflets mounted inside the frame. The leaflets may be attached to the frame along their cusp edge portions and at commissure tabs (also referred to as leaflet tabs) of the leaflets. Mechanically expandable valves, as well as some balloon-expandable valves and self-expandable valves, elongate axially when radially compressed for delivery into a patient. This can cause overstretching of the leaflets in an axial direction, which can deform and/or damage the leaflets.

US 2018/0325665 describes a prosthetic heart valve which includes an annular frame including a plurality of strut members that is radially collapsible and expandable. A leaflet structure is situated within the frame, and includes a plurality of leaflets having opposing commissure tab portions on opposite sides of the leaflet. US 2009/0157175 describes an implantable prosthetic valve which has an upper frame section and a lower frame section. The upper frame section has a plurality of struts and a first leaflet receiving surface at a lower portion of the upper frame section. The lower frame section has a second leaflet receiving surface at an upper portion of the lower frame section. An edge of a flexible leaflet is disposed between the first and second leaflet receiving surfaces to attach the leaflet to the upper and lower frame sections.

Accordingly, a need exists for improved prosthetic heart valve leaflet assemblies, and methods for assembling prosthetic heart valves.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

In a first aspect of the presently claimed invention, a prosthetic heart valve comprises an annular frame that is radially compressible and expandable between a radially compressed state and a radially expanded state, wherein the frame elongates in an axial direction when radially compressed from the radially expanded state to the radially compressed state; and a plurality of leaflets disposed inside of the frame and configured to permit blood to flow through the frame in a first direction and block the flow of blood in a second direction, opposite the first direction. The leaflets are coupled to the frame such that when the frame is in the radially expanded state, the leaflets have slack in an axial direction, wherein when the frame is radially compressed to a partially radially compressed state between the radially expanded state and the radially compressed state, the leaflets are in a relaxed state, and wherein when the frame is in the radially compressed state, the leaflets are stretched in the axial direction.

Also disclosed herein is a prosthetic heart valve which comprises an annular frame that is radially compressible and expandable between a radially compressed state and a radially expanded state, wherein the frame elongates in an axial direction when radially compressed from the radially expanded state to the radially compressed state; and a leaflet assembly comprising a plurality of leaflets connected to each other at commissures of the leaflet assembly, wherein the leaflets are configured to permit blood to flow through the frame in a first direction and block the flow of blood in a second direction, opposite the first direction. Each commissure is coupled to the frame and each leaflet has a cusp edge portion that is coupled to the frame, and wherein each leaflet has two opposing neck regions unattached to the frame, each neck region extending from one of the commissures to an adjacent end of the cusp edge portion. When the frame is in the radially expanded state, the leaflets have slack in an axial direction in the neck regions of the leaflets, wherein when the frame is radially compressed to a partially radially compressed state between the radially expanded state and the radially compressed state, the leaflets are in a relaxed state, and wherein when the frame is in the radially compressed state, the leaflets are stretched in the axial direction along the neck regions.

In a second aspect of the presently claimed invention, a method of assembling a prosthetic heart valve comprises placing a plurality of leaflets in an annular frame that is radially compressible and expandable between a radially compressed state and a radially expanded state, wherein the frame elongates in an axial direction when radially compressed from the radially expanded state to the radially compressed state; placing the frame in a partially radially compressed state between the radially expanded state and the radially compressed state; and coupling the leaflets to the frame while the leaflets are in a relaxed state and the frame is in the partially radially compressed state such that when the frame is expanded to the radially expanded state, the leaflets have slack in an axial direction, and wherein when the frame is compressed to the radially compressed state, the leaflets are stretched in the axial direction.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of an exemplary embodiment of a prosthetic heart valve.
FIG. 2 is a plan view of one of the leaflets of the prosthetic valve of FIG. 1 in a laid flat configuration.
FIGS. 3A-3B are side views of the frame of the prosthetic valve of FIG. 1 in a radially compressed state and a radially expanded state, respectively.
FIG. 4 is a plan view of an alternative embodiment of a leaflet for a prosthetic heart valve.
FIG. 5 is a perspective view of a portion of another embodiment of a prosthetic heart valve that includes leaflets of the type shown in FIG. 4.
FIG. 6A is a schematic cross-sectional view of the prosthetic heart valve of FIG. 5 in a radially compressed state.
FIG. 6B is a schematic cross-sectional view of the prosthetic heart valve of FIG. 5 in a partially radially compressed state.
FIG. 6C is a schematic cross-sectional view of the prosthetic heart valve of FIG. 5 in a radially expanded state.
FIGS. 7A-7B are top plan views of the prosthetic heart valve of FIG. 5 shown with the leaflets in the open position and the closed position, respectively.
FIG. 8 shows a leaflet sub-assembly comprising a leaflet and a discrete skirt secured to the leaflet.
FIG. 9 shows a reinforcing strip for the leaflet sub-assembly of FIG. 8.
FIG. 10 is a perspective view of the leaflet sub-assembly of FIG. 8 after attaching the reinforcing strip to the leaflet.
FIG. 11 is a perspective view of a portion of another embodiment of a prosthetic heart valve that includes leaflet sub-assemblies of the type shown in FIG. 10.
FIG. 12 is an enlarged, cross-sectional view of a portion of the prosthetic heart valve of FIG. 11.
FIG. 13 shows a delivery apparatus adapted to deliver a prosthetic heart valve.

### DETAILED DESCRIPTION

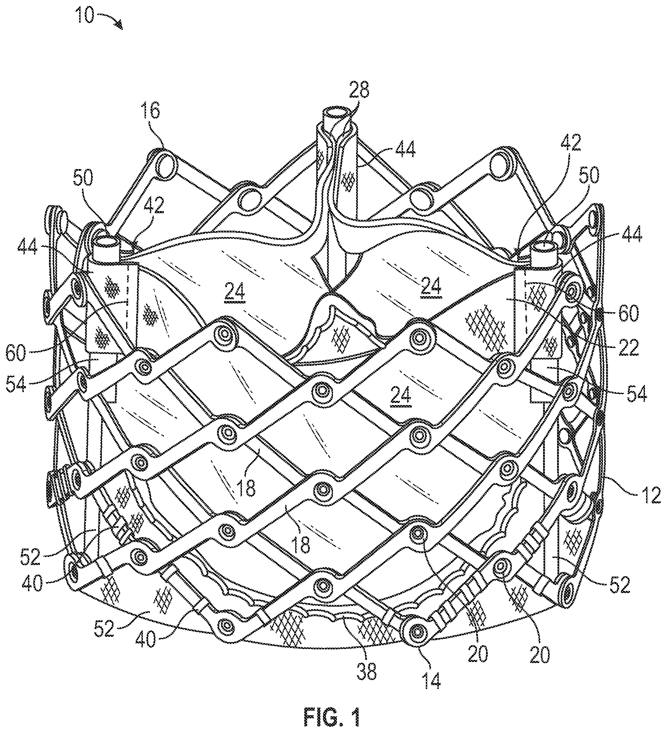

FIG. 1 shows an exemplary prosthetic heart valve 10, according to one embodiment. The prosthetic heart valve 10 is radially compressible and expandable between a radially compressed configuration for delivery into a patient and a radially expanded configuration. In particular embodiments, the prosthetic heart valve 10 can be implanted within the native aortic annulus, although it also can be implanted at other locations in the heart, including within the native mitral valve, the native pulmonary valve, and the native tricuspid valve. The prosthetic heart valve 10 includes an annular stent or frame 12 having a first end 14 and a second end 16.

In the depicted embodiment, the first end 14 is an inflow end and the second end 16 is an outflow end. The outflow end 16 can be coupled to a delivery apparatus for delivering and implanting the prosthetic heart valve within the native aortic valve is a transfemoral, retrograde delivery approach. Thus, in the delivery configuration of the prosthetic heart valve, the outflow end 16 is the proximal-most end of the prosthetic valve. In other embodiments, the inflow end 14 can be coupled to the delivery apparatus, depending on the particular native valve being replaced and the delivery technique that is used (e.g., trans-septal, transapical, etc.). For example, the inflow end 14 can be coupled to the delivery apparatus (and therefore is the proximal-most end of the prosthetic heart valve in the delivery configuration) when delivering the prosthetic heart valve to the native mitral valve via a trans-septal delivery approach.

The frame 12 can be made of any of various suitable materials, such as stainless steel, a cobalt chromium alloy, or a nickel titanium alloy ("NiTi"), for example Nitinol. Referring again to FIG. 1, as shown, the frame 12 can include a plurality of interconnected struts 18 arranged in a lattice-type pattern. The struts 18 are shown as positioned diagonally, or offset at an angle relative to, and radially offset from, a longitudinal axis of the prosthetic heart valve 10 when the prosthetic heart valve 10 is in the expanded configuration. In other implementations, the struts 18 can be offset by a different amount than depicted in FIG. 1, or some or all of the struts 18 can be positioned parallel to the longitudinal axis of the prosthetic heart valve 10.

In the illustrated embodiment, the struts 18 are pivotably coupled to one another at one or more pivot joints along the length of each strut. For example, in the illustrated configuration, each of the struts 18 can be formed with apertures at opposing ends of the strut and apertures spaced along the length of the strut. Respective hinges can be formed at the locations where struts 18 overlap each other via fasteners or pivot members, such as rivets or pins 20 that extend through the apertures. The hinges can allow the struts 18 to pivot relative to one another as the frame 12 is radially expanded or compressed, such as during assembly, preparation, or implantation of the prosthetic heart valve 10.

In some embodiments, the frame 12 can be constructed by forming individual components (e.g., the struts and fasteners of the frame) and then mechanically assembling and connecting the individual components together. In other embodiments, the struts 28 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube). Further details regarding the construction of the frame and the prosthetic heart valve are described in U.S. Patent Application Publication Nos. 2018/0153689, 2018/0344456, and 2019/0060057.

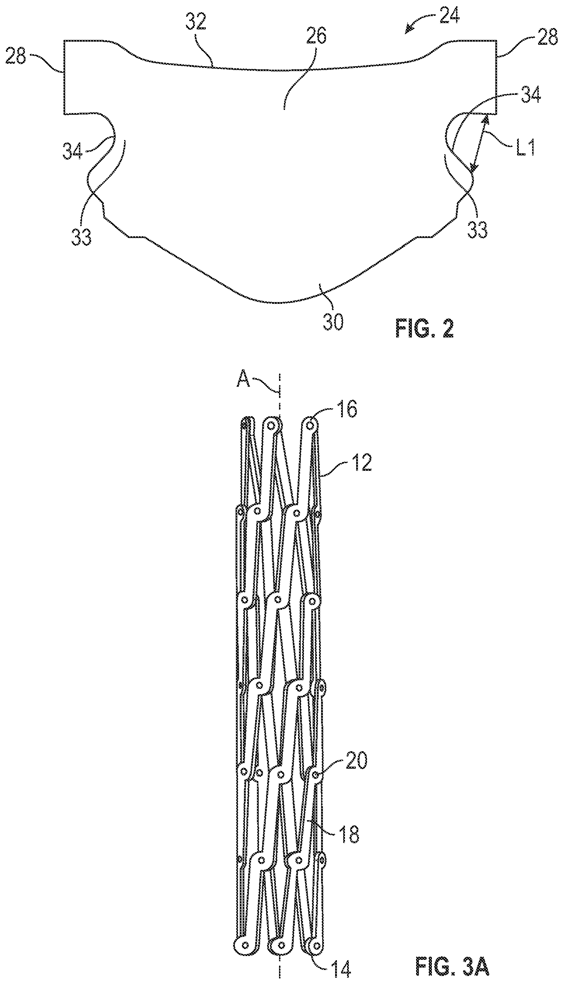

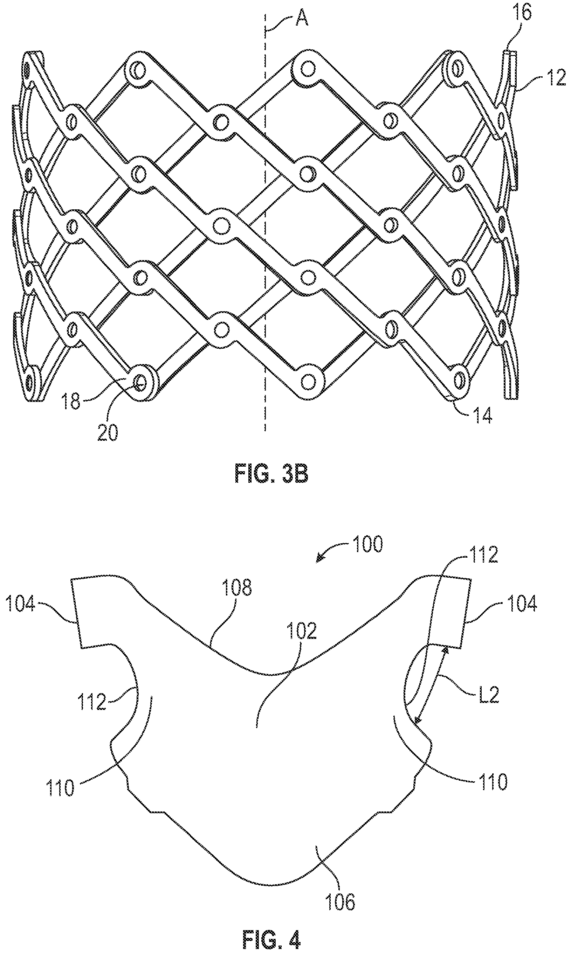

The frame 12 can be radially compressed and expanded between a radially compressed state and a radially expanded state. FIG. 3A shows the bare frame 12 (without other components of the prosthetic valve) is the radially compressed state for delivery into a patient. FIG. 3B shows the bare frame 12 in the radially expanded state, such as when expanded inside the patient's body at a desired implantation site. As shown in FIGS. 3A and 3B, the frame 12 elongates axially along a central longitudinal axis A when the frame is radially compressed.

The prosthetic heart valve 10 also includes a valvular structure 22 which is coupled to the frame 12 and configured to regulate the flow of blood through the prosthetic heart valve 10 from the inflow end 14 to the outflow end 16. The valvular structure 22 includes a leaflet assembly comprising a plurality of leaflets 24 (three leaflets 24 in the illustrated embodiment) made of a flexible material. The leaflets 24 of the leaflet assembly can be made from in whole or part, biological material, bio-compatible synthetic materials, or other such materials. Suitable biological material can include, for example, bovine pericardium (or pericardium from other sources).

As best shown in FIG. 2, each leaflet 24 in the illustrated embodiment includes a main body 26 (also referred to as a "belly" portion of the leaflet), and two opposing commissure tabs 28 arranged on opposite sides of the main body 26. The main body 26 has a cusp edge portion 30 (also referred to as the inflow edge portion of the leaflet) and a free edge portion 32 that can coapt with the free edge portions of adjacent leaflets when the leaflet assembly is in a closed position under pressure from the backflow of blood. As further shown in FIG. 2, in particular embodiments, the main body 26 of each leaflet 24 optionally can include a notch or indention 34 on each side of the leaflet between each commissure tab 28 and an adjacent upper end of the cusp edge portion 30. The portions of the main body containing the notches 34 can be referred to as "neck" regions 33 of the leaflet.

The leaflets 24 can be mounted to the frame in a variety of ways. In the illustrated embodiment, for example, the cusp edge portion 30 of each leaflet can be sutured to an inner skirt 36 along a stitch line 38 (sometimes referred to as a "scallop" line). The stitch line 38 can be formed by forming in-an-out stitches through the cusp edge portions of the leaflets and the skirt with one or more sutures, such as an Ethibond suture. The skirt 36 in turn can be connected to the frame 12 with sutures 40 that extend through the skirt and around selected struts 18 of the frame 12.

Each commissure tab 28 of a leaflet 24 can be paired with an adjacent commissure 28 of an adjacent leaflet 24 to form a commissure 42. The commissures 42 can be mounted to respective commissure support portions of the frame 12. In some embodiments, as shown in FIG. 1, the commissures 42 can be mounted (e.g., sutured) directly to commissure support portions of actuators 50 of the frame 12 via commissure attachment members 44, which can be pieces of relatively tear resistant material, such as a fabric. As one example, as depicted in FIG. 1, at each commissure 42, the commissure tabs 28 can be at least partially wrapped around opposite sides of the commissure support portion and the commissure attachment member 44 can be wrapped around the commissure tabs 28 and the commissure support portion. The commissure attachment member 44 can be stitched to the commissure tabs 28 with sutures 60, thereby securing the commissure 42 to the commissure support portion of the actuator 50.

The neck regions 33 of the leaflets can remain unattached to the frame to promote stretching of the leaflets in the axial direction (along longitudinal axis A) when the frame 12 is radially compressed. The notches 34 are shaped to promote stretching of the leaflets in a radial direction when the frame 12 is radially expanded to its functional size.

In other embodiments, the commissures 42 can be mounted to support struts or posts of the frame that are separate from the actuators 50. In still other embodiments, the commissures 42 can be secured to support struts or posts that are integral components of the frame. Further details regarding transcatheter prosthetic heart valves, including the manner in which the valvular structure can be coupled to the frame 12 of the prosthetic heart valve 10, can be found, for example, in U.S. Patent Nos. 6,730,118, 7,393,360, 7,510,575, 7,993,394, and 8,652,202, and U.S. Patent Application Publication No. 2018/0325665.

As noted above, the prosthetic heart valve 10 can further include a plurality of actuators 50, which are mounted to and equally spaced around the inner surface of the frame 12. The actuators are configured to apply expansion and compression forces to the frame for radially expanding and compressing the prosthetic valve.

In the illustrated embodiment, the actuators 50 are linear actuators, each of which comprises an inner member, or piston, 52 and an outer member, or cylinder, 54. The inner member 52 is pivotably coupled to a junction of the frame, such as at the first end 14, while the outer member 54 is pivotably coupled to another junction of the frame closer to the second end 16. Moving the inner member 52 proximally relative to the outer member 54 and/or moving the outer member 54 distally relative to the inner member 52 is effective to radially expand the prosthetic valve. Conversely, moving the inner member 52 distally relative to the outer member 54 and/or moving the outer member 54 proximally relative to the inner member 52 is effective to radially compress the prosthetic valve. The actuators 50 can include locking mechanisms, such as ratchet mechanisms, that are configured to retain the prosthetic valve in an expanded state inside the patient's body.

In some embodiments, each of the actuators 50 can be configured to form a releasable connection with one or more respective actuators of a delivery apparatus of a transcatheter delivery system. The actuators of the delivery apparatus can transmit forces from a handle of the delivery apparatus to the actuators 50 for expanding or compressing the prosthetic valve. Further details of the actuators, locking mechanisms and delivery apparatuses for actuating the actuators can be found in U.S. Patent Application Publication Nos. 2018/0153689, 2019/0060057 and 2018/0325665. Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

As noted above, each of the actuators 50 can be used to support a respective commissure 42 of the leaflet assembly. In the illustrated embodiment, for example, the commissures 42 are mounted on the outer members 54 of the actuators, which serve as commissure support portions of the prosthetic valve.

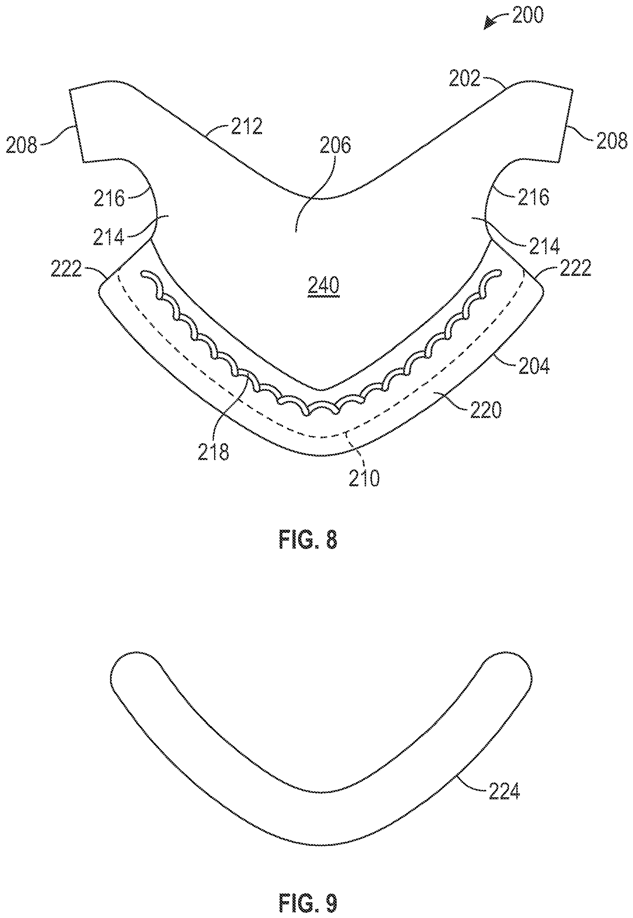

The prosthetic heart valve 10 can also include one or more skirts or sealing members. For example, as shown in FIG. 1, the prosthetic heart valve 10 can include an inner skirt 36 mounted on the inner surface of the frame 12. As shown in FIG. 1, the inner skirt 36 is a circumferential inner skirt that spans an entire circumference of the inner surface of the frame 12. The inner skirt 36 can function as a sealing member to prevent or decrease perivalvular leakage (e.g., when the valve is placed at the implantation site) and as an attachment surface to anchor the leaflets 24 to the frame 12. For example, as discussed above, the cusp edge portions 30 of the leaflets 24 can be sutured directly to the inner skirt 36, which in turn can be directly connected to selected struts 18 of the frame, such as with sutures 40, as shown in FIG. 1. In alternative embodiments, each leaflet 24 can be sutured to a discrete skirt, which is turn is connected to selected struts 18 of the frame, as further described below in connection with FIGS. 8-12).

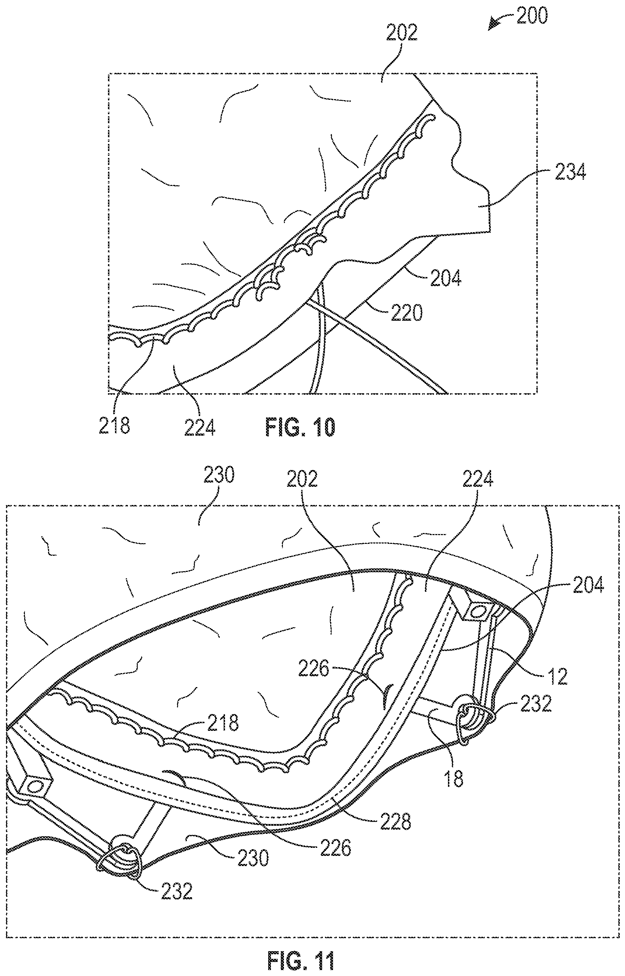

The prosthetic heart valve 10 can also include an outer skirt mounted on the outer surface of the frame 12 (not shown in FIG. 1; see, e.g., outer skirt 230 in FIG. 11). The outer skirt can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials (e.g., polyethylene terephthalate (PET)) or natural tissue (e.g., pericardial tissue). In particular embodiments, the skirts can be formed from any of various biocompatible fabrics (e.g., a PET fabric), which can have woven, braided, or knitted construction or combinations thereof. The inner and outer skirts can be mounted to the frame using sutures, an adhesive, welding, and/or other means for attaching the skirts to the frame.

FIG. 2 shows a leaflet 24 in a non-deformed, or "free state", meaning that the leaflet is not stretched axially or radially. Typically, the leaflets of a conventional prosthetic valve are sized and shaped relative to the frame to assume a non-deformed state when the frame is in a radially expanded state, which is desirable to promote coaptation of the free edge portions 32 of the leaflets 24 when the leaflet assembly is in a closed state blocking the flow of the blood through the prosthetic valve. In some cases, the frame elongates axially when the frame is radially compressed from the radially expanded state to the radially compressed state, thereby stretching the leaflets in the axial direction. As depicted in FIGS. 3A-3B, in some embodiments, the length of the frame 12 can increase by 100% when radially compressed from the fully expanded state (FIG. 3B) to the fully compressed state (FIG. 3A). This increase in the length of the frame can overstretch the leaflets, which can damage the leaflets.

Thus, in particular embodiments, the leaflets 24 are sized and shaped relative to the frame 12 to assume a non-deformed or free state when the frame is in an intermediate state between the fully compressed state and the fully expanded state. The intermediate state is referred to as a partially radially compressed state of the frame. This reduces stretching of the leaflets in the axial direction when the frame is radially compressed to the fully compressed state. In particular, the amount of stretching of the neck regions 33 (which are unattached to the frame) in the axial direction is reduced, thereby avoiding or minimizing damage to the leaflets when the frame is radially compressed.

In particular embodiments, a prosthetic valve, such as prosthetic valve 10, can include a frame of a first size (equal to an expanded diameter of the frame) and leaflets that are designed for a frame of a second, smaller size (equal to an expanded diameter of the frame), which allows the leaflets to assume a free state when the frame is in a partially radially compressed state. In other words, the leaflets designed for a prosthetic valve of a first size (e.g., 23 mm) can be assembled in a frame of a prosthetic valve of a second, larger size (e.g., 29 mm).

FIG. 4 shows an example of a leaflet 100 that has a slightly different configuration than the leaflet 24 and is designed for a relatively smaller size prosthetic valve. Like leaflet 24, the leaflet 100 has a main body 102, commissure tabs 104, a cusp edge portion 106, a free edge portion 108, and neck regions 110, which can define notches 112. Because the leaflet 100 is designed for a relatively smaller size valve, the notches 112 of neck regions 110 can have a length L2 that is longer than a length L1 of notches 34 of neck regions 33 of the leaflet 24. Further, the free edge portion 108 of the leaflet 100 can a more pronounced V-shape compared to the free edge portion 32 of the leaflet 24.

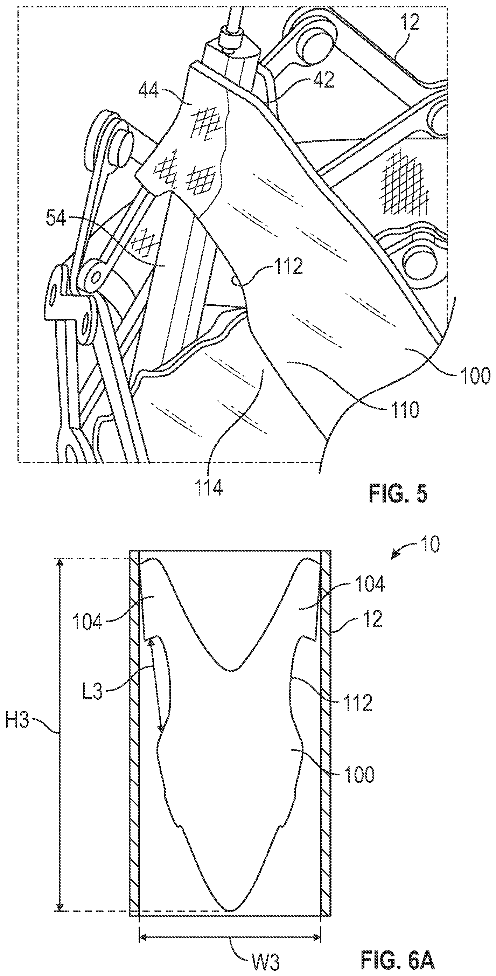

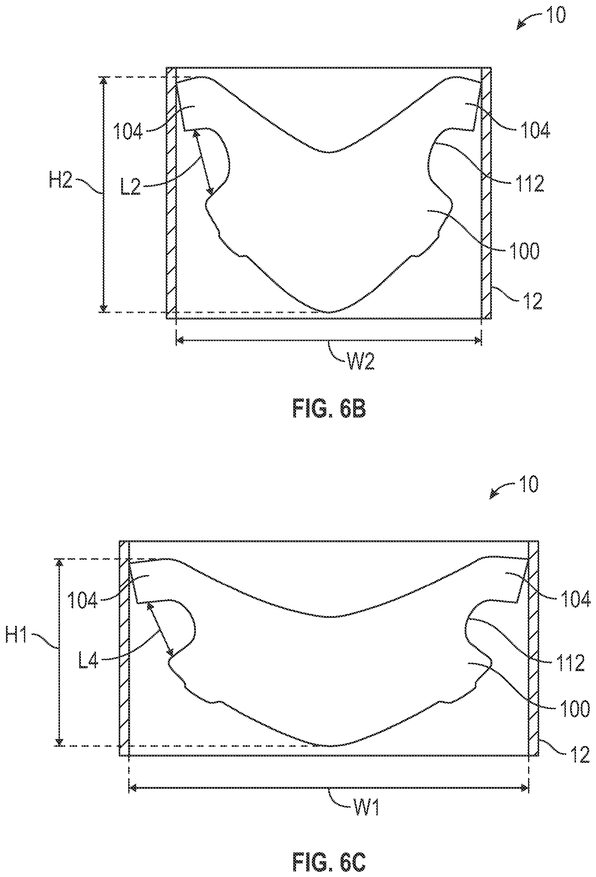

In some embodiments, as shown in FIG. 5, a prosthetic valve 10 can include a plurality of leaflets 100 (e.g., three) instead of leaflets 24. FIGS. 6A-6C are schematic representations of the prosthetic valve 10 with leaflets 100 (one which is shown for purposes of illustration) in various stages of compression. FIG. 6A shows the prosthetic valve 10 in a fully radially compressed state. FIG. 6B shows the prosthetic valve 10 is a partially radially compressed state. FIG. 6C show the prosthetic valve 10 in a fully expanded state.

The leaflets 100 are in a free state in the partially radially compressed state of FIG. 6C. Thus, when the prosthetic valve 10 is in the fully expanded state (FIG. 6C), the leaflets 100 have a height H1 (measured from the inflow-most location of the leaflet to the outflow-most location of the leaflet) and a width W1 (measured from the radial outermost edge of a commissure tab 104 to the same location on the opposing tab 104). When the prosthetic valve 10 is in the partially compressed state (FIG. 6B), the leaflets 100 have a height H2 that is greater than H1 and width W2 that is less than W1. When the prosthetic valve is in the fully compressed state (FIG. 6A), the leaflets 100 have a height H3 that is greater than H2 and a width W3 that is less than W2. Further, the length of the notches 112 increase from L2 in the partially compressed state to L3 in the fully compressed state. The length of notches 112 can decrease from L2 in the partially compressed state to L4 in the fully expanded state.

As best shown in FIG. 5, when the prosthetic valve 10 is in the fully expanded state, slack 114 can form along the neck regions 110 as a result of the decreased height H1 of the leaflet. The slack 114 forms in an axial direction, meaning that portions of the leaflet are brought closer together in an axial direction to form the slack. When the portions of the leaflets are brought closer together, they can a radially extending fold in the neck regions 110. Moreover, when the prosthetic valve 10 is in the fully expanded state, the leaflets 100 can be stretched in a transverse plane perpendicular to the central longitudinal axis of the prosthetic valve. In other words, the width W1 of the leaflet 100 (FIG. 6C) can represent the width of the leaflet that is stretched in a widthwise direction of the leaflet extending from one commissure tab 104 to the other commissure 104.

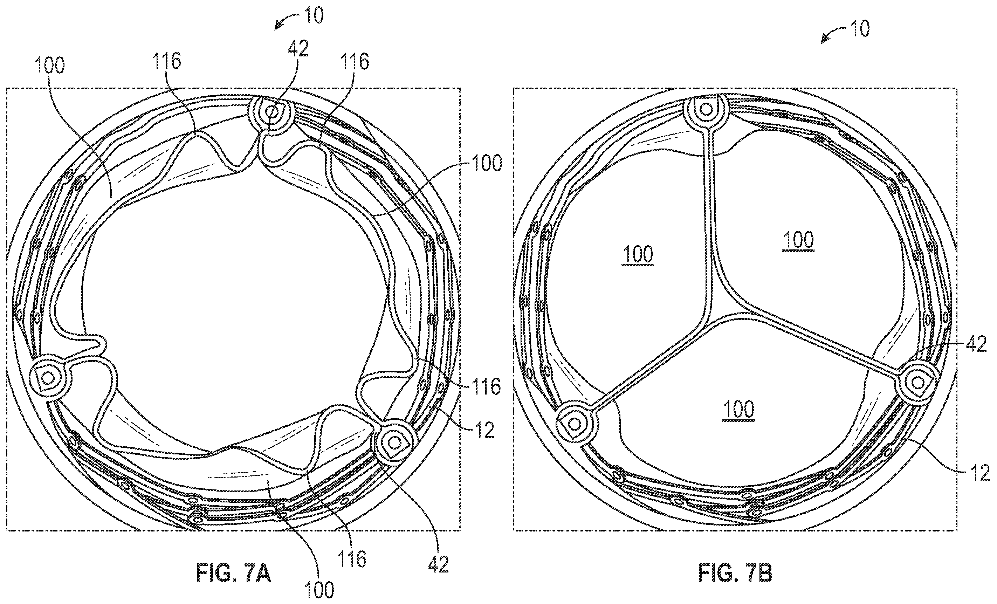

FIGS. 7A and 7B show the prosthetic valve 10 with leaflets 100 in an open state and a closed state, respectively. In the open state of the leaflets (FIG. 7A), slack 116 can form along the free edge portions of the leaflets 100 in the vicinity of the commissures 42. The slack 116 can form axially extending folds extending downwardly from the free edge portions of the leaflets.

In one specific implementation, the prosthetic valve 10 can include a frame 12 for a 29-mm valve (the size of the valve being the outer diameter of the frame in the radially expanded state) and leaflets 100 for a 23-mm valve. The notches 112 of the leaflets 100 can have a length L2 of about 7 mm in its free state (FIG. 6B), a length L3 of about 10 mm when the frame is fully radially compressed (FIG. 6A), and a length L4 of about 5 mm to 7 mm when the frame is fully radially expanded (FIG. 6C). Comparatively, the same prosthetic valve 10 having leaflets 24 sized for a 29-mm valve (where the leaflets are in a free state when the frame is fully radially expanded), the notches 34 stretch axially from a length L1 of about 5 mm when the frame is fully expanded to about 10 mm when the frame is fully compressed. As such, it can be appreciated that the leaflets 100 undergo less stretching in an axial direction when the frame is fully radially compressed than leaflets 24.

It should be noted that leaflet 100 is one example of a leaflet that can be used in the prosthetic valve 10 to reduce axial stretching of the leaflet when the frame is fully radially compressed. In particular, the prosthetic valve can have leaflets that have the same overall shape of leaflets 24 (or other shapes) but are otherwise sized such that the leaflets are in a free state when the frame is in a partially radially compressed state.

As discussed above, in the embodiment of FIG. 1, the cusp edge portions 30 of the leaflets 24 are sutured to an inner skirt 36. Since the inner skirt 36 spans the entire circumference of the inner surface of the frame 12, attachment of numerous leaflets (for example, three leaflets) 24 to the inner skirt 36 is time consuming and requires delicate assembly skills to avoid defects that might occur through the assembly process.

Thus, it may be desirable to assemble each leaflet of a leaflet assembly separately, with an individual or discrete skirt and leaflet. For example, a leaflet assembly may include a plurality of separately formed leaflet sub-assemblies, each leaflet sub-assembly including a skirt and leaflet secured to one another along a cusp edge portion of the leaflet. In some embodiments, the skirt and an additional reinforcing element may form a supporting structure for the leaflet. Each individual leaflet sub-assembly may then be connected to additional leaflet sub-assemblies at their commissure tabs to form commissures. The leaflet assembly can then be placed inside a frame. The skirt and optionally the reinforcing element of each of each leaflet sub-assembly can be connected (e.g., via sutures) to struts of the frame and/or an outer skirt. The commissures can be connected to respective commissure support members (e.g., actuators 50) as disclosed herein. Such an assembly process may allow each leaflet sub-assembly to be assembled more quickly and easily, on a relatively flat, two-dimensional platform, prior to being attached to the three-dimensional frame of the prosthetic heart valve.

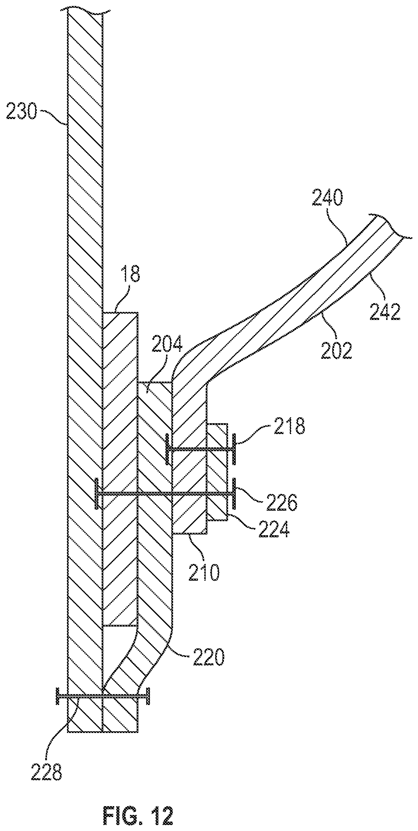

FIG. 8 shows a leaflet sub-assembly 200, according to one embodiment, that can be used in the assembly of a prosthetic valve, such as prosthetic valve 10. The leaflet sub-assembly 200 in the illustrated embodiment includes a leaflet 202 and a discrete skirt 204. Like leaflets 24 and 100, the leaflet 202 has a main body 206, commissure tabs 208, a cusp edge portion 210, a free edge portion 212, and neck regions 214, which can define notches 216. The leaflet 202 has first and second major surfaces 240, 242, respectively (see FIG. 12). When assembled in a frame, the first major surface 240 is an outwardly facing surface and also the outflow surface or outflow side of the leaflet. The second major surface 242 is an inwardly facing surface and also the inflow surface or inflow side of the leaflet.

The skirt 204 can be connected to the outwardly facing surface 240 of the cusp edge portion 210 with a suture 206 which can track the curvature of the cusp edge portion 210 and the skirt 204. A lower portion of the skirt 204 can extend beyond the cusp edge portion 210 of the leaflet to form an extended portion 220 which can be used to connect the skirt 204 to other components of the prosthetic valve. As further shown in FIG. 8, the skirt 204 desirably is sized and shaped such that opposite ends 222 of the skirt 204 are aligned or substantially aligned with the opposite ends of the cusp edge portion 210 of the leaflet and do not extend into the notches 216. In this manner, the skirt 204 does not inhibit the leaflet from stretching in the axial direction along the neck regions 214 when the frame 12 is radially compressed to the radially compressed state.

An exemplary method, useful for understanding the presently claimed invention, and for assembling a prosthetic heart valve, such as prosthetic valve 10 is as follows. A plurality of leaflet sub-assemblies 200 can be formed in the manner described above and shown in FIG. 8 for each leaflet of the prosthetic valve. For example, three leaflet sub-assemblies 200 can be formed if the prosthetic valve is to have three leaflets 202. Each leaflet sub-assembly 200 can then be positioned into a frame 12. The skirt 204 of each leaflet sub-assembly 200 can be connected to the frame 12, such as by suturing each skirt 204 to selected struts 18 (similar to the way skirt 36 is sutured to struts 18) and/or suturing the skirt 204 to an outer skirt (such as outer skirt 230, described below).

The commissures tabs 208 can be connected to adjacent commissure tabs 208 of adjacent leaflets to form commissures (similar to commissures 42), which are then connected to respective commissure support members of the frame, such as actuators 50. Commissure attachment members (e.g., attachment members 44) can be used to attach the commissures to the actuators 50 or other commissure support members of the frame. In some embodiments, the commissures are at least partially assembled or fully assembled prior to placing the leaflet sub-assemblies 200 inside the fame and connecting the skirts 204 to the frame, such as disclosed in U.S. Provisional Application No. 62/928,993, filed October 31, 2019.

Referring to FIGS. 9-10, in some embodiments, each leaflet sub-assembly 200 can further include a discrete reinforcing strip 224 secured to the inwardly facing surface 242 of the cusp edge portion 210, on the opposite side of the leaflet from the skirt 204. The reinforcing strip 224 can be secured to the leaflet 202 and the skirt 204 with the suture 218. The opposing end portions of the reinforcing strip 224 can include projections or wing portions 234 (one of which is shown in FIG. 10). The wing portions 234 can be secured to respective actuators (e.g., actuators 50 of FIG. 1), such as with sutures, to help seal the sub-commissure area of the leaflet assembly during valve cycling.

The skirts 204 and reinforcing strips 224 can be formed from any of various suitable biocompatible materials, including any of various synthetic materials (e.g., (PET) or natural tissue (e.g., pericardial tissue). In particular embodiments, the skirts 204 and reinforcing strips 224 can be formed from any of various biocompatible fabrics (e.g., a PET fabric), which can have woven, braided, or knitted construction or combinations thereof.

Referring now to FIGS. 11-12, after forming each leaflet sub-assembly 200 including a reinforcing strip 224, and optionally pre-assembling the commissures, the cusp edge portions of each sub-assembly 200 can be connected to selected struts 18 of the frame 12 with sutures 226, each of which can extend through the skirt 204, the cusp edge portion 210 of the leaflet, and the reinforcing strip 224 and around a selected strut 18. In some embodiments, one or more of the sutures 226 can be placed through the skirt 204 around a selected strut 18 while avoiding the cusp edge portion 210 and the reinforcing strip 224.

In particular embodiments, as shown in FIGS. 11-12, each skirt 204 can be further connected to an outer skirt 230 of the prosthetic valve with one or more sutures 228. The sutures 228 can be attached in the form of in-and-out stitches that extend through the skirts 204 and the outer skirt 230 along an undulating stitch line that tracks the curvature of the skirts 204. The outer skirt 230 can be an annular skirt that extends completely around an outer surface of the frame 12. The outer skirt 230 can be connected to the frame 12, such as with sutures 232 that extend around selected struts and through the outer skirt 230. Further details regarding the outer skirt are disclosed in U.S. Application No. 16/252,890 and 62/797,837.

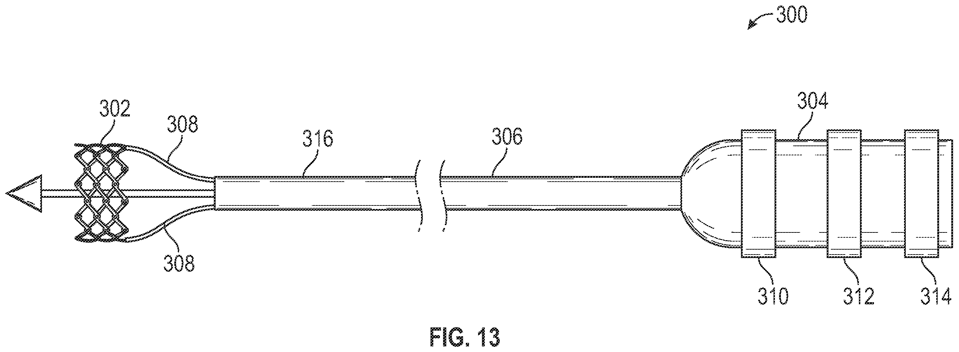

FIG. 13 illustrates a delivery apparatus 300, according to one embodiment, adapted to deliver a prosthetic heart valve 302, such as the illustrated prosthetic heart valve 10, described above. The prosthetic valve 302 can be releasably coupled to the delivery apparatus 300. It should be understood that the delivery apparatus 300 and other delivery apparatuses disclosed herein can be used to implant prosthetic devices other than prosthetic valves, such as stents or grafts.

The delivery apparatus 300 in the illustrated embodiment generally includes a handle 304, a first elongated shaft 306 (which comprises an outer shaft in the illustrated embodiment) extending distally from the handle 304, at least one actuator assembly 308 extending distally through the outer shaft 306. The at least one actuator assembly 308 can be configured to radially expand and/or radially collapse the prosthetic valve 302 when actuated.

Though the illustrated embodiment shows two actuator assemblies 308 for purposes of illustration, it should be understood that one actuator 308 can be provided for each actuator on the prosthetic valve. For example, three actuator assemblies 308 can be provided for a prosthetic valve having three actuators (e.g., actuators 50). In other embodiments, a greater or fewer number of actuator assemblies can be present.

In some embodiments, a distal end portion 316 of the shaft 306 can be sized to house the prosthetic valve in its radially compressed, delivery state during delivery of the prosthetic valve through the patient's vasculature. In this manner, the distal end portion 316 functions as a delivery sheath or capsule for the prosthetic valve during delivery,

The actuator assemblies 308 can be releasably coupled to the prosthetic valve 302. For example, in the illustrated embodiment, each actuator assembly 308 can be coupled to a respective actuator (e.g., actuator 50) of the prosthetic valve 302. Each actuator assembly 308 can comprise a support tube, an actuator member, and a locking tool. The actuator member can extend through the support tube. The support tube has a distal end portion that can be coupled to the outer member 54 of a respective actuator 50, and the actuator member has a distal end portion that can be coupled to the inner member 52 of the respective actuator 50. When actuated, the actuator assembly 308 can transmit pushing and/or pulling forces to the inner and outer members 52, 54 to radially expand and collapse the prosthetic valve as previously described. The actuator assemblies 308 can be at least partially disposed radially within, and extend axially through, one or more lumens of the outer shaft 306. For example, the actuator assemblies 308 can extend through a central lumen of the shaft 306 or through separate respective lumens formed in the shaft 306.

The handle 304 of the delivery apparatus 300 can include one or more control mechanisms (e.g., knobs or other actuating mechanisms) for controlling different components of the delivery apparatus 300 in order to expand and/or deploy the prosthetic valve 302. For example, in the illustrated embodiment the handle 304 comprises first, second, and third knobs 310, 312, and 314.

The first knob 310 can be a rotatable knob configured to produce axial movement of the outer shaft 306 relative to the prosthetic valve 302 in the distal and/or proximal directions in order to deploy the prosthetic valve from the delivery sheath 316 once the prosthetic valve has been advanced to a location at or adjacent the desired implantation location with the patient's body. For example, rotation of the first knob 310 in a first direction (e.g., clockwise) can retract the sheath 316 proximally relative to the prosthetic valve 302 and rotation of the first knob 310 in a second direction (e.g., counter-clockwise) can advance the sheath 316 distally. In other embodiments, the first knob 310 can be actuated by sliding or moving the knob 310 axially, such as pulling and/or pushing the knob. In other embodiments, actuation of the first knob 310 (rotation or sliding movement of the knob 310) can produce axial movement of the actuator assemblies 308 (and therefore the prosthetic valve 302) relative to the delivery sheath 316 to advance the prosthetic valve distally from the sheath 316.

The second knob 312 can be a rotatable knob configured to produce radial expansion and/or contraction of the prosthetic valve 302. For example, rotation of the second knob 312 can move the actuator member and the support tube axially relative to one another. Rotation of the second knob 312 in a first direction (e.g., clockwise) can radially expand the prosthetic valve 302 and rotation of the second knob 112 in a second direction (e.g., counter-clockwise) can radially collapse the prosthetic valve 302. In other embodiments, the second knob 312 can be actuated by sliding or moving the knob 312 axially, such as pulling and/or pushing the knob.

The third knob 314 can be a rotatable knob configured to retain the prosthetic heart valve 302 in its expanded configuration. For example, the third knob 314 can be operatively connected to a proximal end portion of the locking tool of each actuator assembly 308. Rotation of the third knob in a first direction (e.g., clockwise) can rotate each locking tool to advance the locking nuts to their distal positions to resist radial compression of the frame of the prosthetic valve, as described above. Rotation of the knob 314 in the opposite direction (e.g., counterclockwise) can rotate each locking tool in the opposite direction to decouple each locking tool from the prosthetic valve 302. In other embodiments, the third knob 314 can be actuated by sliding or moving the third knob 314 axially, such as pulling and/or pushing the knob.

Although not shown, the handle 304 can include a fourth rotatable knob operative connected to a proximal end portion of each actuator member. The fourth knob can be configured to rotate each actuator member, upon rotation of the knob, to unscrew each actuator member from the proximal portion of a respective actuator. As described above, once the locking tools and the actuator members are uncoupled from the prosthetic valve 302, they can be removed from the patient.

Other delivery devices can be used to deliver and implant the prosthetic valves disclosed herein, such as those disclosed in PCT Application No. PCT/US2020/063104, filed December 3, 2020, and U.S. Application No. 62/990,299, filed March 16, 2020.

A method of delivering prosthetic valve 302 generally includes placing prosthetic valve 302 in a radially compressed state, e.g., by operating the actuators 50 of the prosthetic valve to place the frame in a radially compressed configuration. This can be accomplished by releasably coupling the prosthetic valve to the actuator assemblies 308 of the delivery device and actuating the second knob 312. The radially compressed prosthetic valve optionally can be placed within a sheath 316 of the delivery device. The delivery device and prosthetic device can be advanced over a guidewire through the vasculature of a patient to a selected implantation site (e.g., the native aortic annulus). For example, when implanting the prosthetic valve within the native aortic valve, the delivery device and prosthetic valve can be inserted into and through a femoral artery, and through the aorta to the native aortic valve. At the implantation site, if initially contained within a sheath 316, the prosthetic valve 302 can be deployed from the sheath 316 by actuating the first knob 310. The prosthetic valve 302 can then be radially expanded to a desired size by actuating the second knob 312. Once the prosthetic device is at the desired diameter, the third knob 314 can be actuated to lock the prosthetic valve at the desired diameter. Thereafter, the actuator assemblies of the delivery device can uncoupled from the prosthetic valve by actuating the fourth knob, allowing removal of the delivery device from the patient's body.

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein, with reference to the prosthetic heart valve and the delivery device or apparatus, "proximal" refers to a position, direction, or portion of a component that is closer to the user and a handle of the delivery apparatus that is outside the patient, while "distal" refers to a position, direction, or portion of a component that is further away from the user and the handle and closer to the implantation site. The terms "longitudinal" and "axial" refer to an axis extending in the proximal and distal directions, unless otherwise expressly defined. Further, the term "radial" refers to a direction that is arranged perpendicular to the axis and points along a radius from a center of an object (where the axis is positioned at the center, such as the longitudinal axis of the prosthetic valve).

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope of these claims.

## Claims

1. A prosthetic heart valve (10) comprising:
an annular frame (12) that is radially compressible and expandable between a radially compressed state and a radially expanded state, wherein the frame (12) elongates in an axial direction when radially compressed from the radially expanded state to the radially compressed state; and
a plurality of leaflets (24) disposed inside of the frame (12) and configured to permit blood to flow through the frame (12) in a first direction and block the flow of blood in a second direction, opposite the first direction;
wherein the leaflets (24) are coupled to the frame (12) such that when the frame is in the radially expanded state, the leaflets have slack (114) in an axial direction, wherein when the frame (12) is radially compressed to a partially radially compressed state between the radially expanded state and the radially compressed state, the leaflets (24) are in a relaxed state, and wherein when the frame (12) is in the radially compressed state, the leaflets (24) are stretched in the axial direction.

2. The prosthetic heart valve of claim 1, wherein the frame (12) comprises a plurality of struts (18) pivotably connected to each other at a plurality of pivot joints, wherein the struts (18) can pivot relative to each other when the frame is radially compressed and expanded.

3. The prosthetic heart valve of any previous claim, wherein each leaflet (24) comprises a cusp edge portion (30) connected to the frame (12) and two opposing commissure tabs (28), wherein each commissure tab (28) is paired with an adjacent commissure tab of an adjacent leaflet (24) to form a commissure (42), which is connected to the frame (12).

4. The prosthetic heart valve of claim 3, wherein each leaflet (24) comprises two opposing neck regions (33), each extending from one of the commissure tabs (28) to an adjacent end of the cusp edge portion (30), wherein the neck regions (33) are unattached to the frame (12) such that the neck regions (33) can stretch in the axial direction when the frame (12) is in the radially compressed state.

5. The prosthetic heart valve of claim 4, wherein neck regions (33) define notches (34) in the leaflets (24).

6. The prosthetic heart valve of any of claims 3-5, further comprising a plurality of actuators (50) connected to the frame (12), wherein the actuators (50) are configured to produce radial expansion and compression of the frame (12).

7. The prosthetic heart valve of claim 6, wherein each commissure (42) is connected to one of the actuators (50).

8. The prosthetic heart valve of any of claims 3-7, further comprising a plurality of fabric skirts (204), each being sutured to the cusp edge portion (30) of a corresponding leaflet (24) and to the frame (12).

9. The prosthetic heart valve of claim 8, wherein each fabric skirt (204) has opposing ends that are aligned with adjacent ends of the cusp edge portion (30) of a corresponding leaflet (24).

10. The prosthetic heart valve of any of claims 8-9, further comprising a plurality of fabric reinforcing strips (224), each being sutured to the cusp edge portion (30) of a corresponding leaflet (24) on an opposite side of the cusp edge portion (30) from the fabric skirt (204) of the same leaflet (24).

11. A method of assembling a prosthetic heart valve (10), the method comprising:
placing a plurality of leaflets (24) in an annular frame (12) that is radially compressible and expandable between a radially compressed state and a radially expanded state, wherein the frame (12) elongates in an axial direction when radially compressed from the radially expanded state to the radially compressed state;
placing the frame (12) in a partially radially compressed state between the radially expanded state and the radially compressed state; and
coupling the leaflets (24) to the frame (12) while the leaflets (24) are in a relaxed state and the frame (12) is in the partially radially compressed state such that when the frame (12) is expanded to the radially expanded state, the leaflets (24) have slack in an axial direction, and wherein when the frame (12) is compressed to the radially compressed state, the leaflets (24) are stretched in the axial direction.

12. The method of claim 11, wherein each leaflet (24) comprises a cusp edge portion (30), two opposing commissure tabs (28), and two opposing neck regions (33), each neck region (33) extending from one of the commissure tabs (28) to an adjacent end of the cusp edge portion (30), wherein each commissure tab (28) is paired with an adjacent commissure tab (28) of an adjacent leaflet (24) to form a commissure (42), and wherein coupling the leaflets (24) to the frame (12) comprises coupling the cusp edge portion (30) of each leaflet (24) to the frame (12) and coupling each commissure (42) to the frame (12) such that the neck regions (33) remain unattached to the frame (12).

13. The method of claim 12, wherein the cusp edge portion (30) of each leaflet (24) is sutured to a skirt (204), which is connected to struts (18) of the frame (12) with sutures (40), each commissure (42) is connected to a commissure support portion of the frame (12), and each neck region (33) defines an axially extending gap between an adjacent commissure (42) and an adjacent end of the cusp edge portion (30) of a corresponding leaflet (24).

14. The method of claim 13, wherein the cusp edge portion (30) of each leaflet (24) is sutured to:
a) a discrete skirt; or
b) the same skirt that extends an entire circumference of an inner surface of the frame (12).

15. The method of claim 13 or claim 14, wherein the cusp edge portion (30) of each leaflet (24) is sutured to a reinforcing strip (224) opposite the skirt (204).

## Patentansprüche

1. Herzklappenprothese (10), umfassend:
einen ringförmigen Rahmen (12), der zwischen einem radial komprimierten Zustand und einem radial expandierten Zustand radial kompressibel und expandierbar ist, wobei der Rahmen (12) sich in einer axialen Richtung längt, wenn er von dem radial expandierten Zustand zu dem radial komprimierten Zustand radial komprimiert wird; und
eine Vielzahl von Segeln (24), die innerhalb des Rahmens (12) angeordnet sind und ausgestaltet sind, um das Strömen von Blut durch den Rahmen (12) hindurch in einer ersten Richtung zuzulassen und das Strömen von Blut in einer zweiten Richtung, die der ersten Richtung entgegengesetzt ist, zu blockieren;
wobei die Segel (24) so an den Rahmen (12) gekoppelt sind, dass, wenn sich der Rahmen in dem radial expandierten Zustand befindet, die Segel in einer axialen Richtung Lose (114) aufweisen, wobei, wenn der Rahmen (12) in einen teilweise radial komprimierten Zustand zwischen dem radial expandierten Zustand und dem radial komprimierten Zustand radial komprimiert wird, die Segel (24) sich in einem relaxierten Zustand befinden, und wobei die Segel (24), wenn sich der Rahmen (12) in einem radial komprimierten Zustand befindet, in der axialen Richtung gestreckt sind.

2. Herzklappenprothese nach Anspruch 1, wobei der Rahmen (12) eine Vielzahl von Streben (18) umfasst, die schwenkbar an einer Vielzahl von Schwenkgelenken miteinander verbunden sind, wobei die Streben (18) relativ zueinander schwenken können, wenn der Rahmen radial komprimiert und expandiert wird.

3. Herzklappenprothese nach einem der vorhergehenden Ansprüche, wobei jedes Segel (24) einen Kuspenrandabschnitt (30), der mit dem Rahmen (12) verbunden ist, und zwei gegenüberliegende Kommissurlaschen (28) umfasst, wobei jede Kommissurlasche (28) mit einer benachbarten Kommissurlasche eines benachbarten Segels (24) gepaart ist, um eine Kommissur (42) zu bilden, die mit dem Rahmen (12) verbunden ist.

4. Herzklappenprothese nach Anspruch 3, wobei jedes Segel (24) zwei gegenüberliegende Halsregionen (33) umfasst, von denen jede sich von einer der Kommissurlaschen (28) zu einem benachbarten Ende des Kuspenrandabschnitts (30) erstreckt, wobei die Halsregionen (33) an dem Rahmen (12) unbefestigt sind, so dass sich die Halsregionen (33) in die axiale Richtung strecken können, wenn sich der Rahmen (12) in dem radial komprimierten Zustand befindet.

5. Herzklappenprothese nach Anspruch 4, wobei Halsregionen (33) Kerben (34) in den Segeln (24) definieren.

6. Herzklappenprothese nach einem der Ansprüche 3 bis 5, des Weiteren umfassend eine Vielzahl von Aktuatoren (50), die mit dem Rahmen (12) verbunden sind, wobei die Aktuatoren (50) ausgestaltet sind, um radiale Expansion und Komprimierung des Rahmens (12) zu erzeugen.

7. Herzklappenprothese nach Anspruch 6, wobei jede Kommissur (42) mit einem der Aktuatoren (50) verbunden ist.

8. Herzklappenprothese nach einem der Ansprüche 3 bis 7, des Weiteren umfassend eine Vielzahl von Textilschürzen (204), von denen jede an den Kuspenrandabschnitt (30) eines entsprechenden Segels (24) und an den Rahmen (12) genäht ist.

9. Herzklappenprothese nach Anspruch 8, wobei jede Textilschürze (204) gegenüberliegende Enden aufweist, die mit benachbarten Enden des Kuspenrandabschnitts (30) eines entsprechenden Segels (24) ausgerichtet sind.

10. Herzklappenprothese nach einem der Ansprüche 8 bis 9, des Weiteren umfassend eine Vielzahl von Textilverstärkungsstreifen (224), von denen jeder an den Kuspenrandabschnitt (30) eines entsprechenden Segels (24) an einer gegenüberliegenden Seite des Kuspenrandabschnitts (30) von der Textilschürze (204) desselben Segels (24) genäht ist.

11. Verfahren zur Montage einer Herzklappenprothese (10), wobei das Verfahren umfasst:
Platzieren einer Vielzahl von Segeln (24) in einem ringförmigen Rahmen (12), der zwischen einem radial komprimierten Zustand und einem radial expandierten Zustand radial kompressibel und expandierbar ist, wobei der Rahmen (12) sich in einer axialen Richtung längt, wenn er von dem radial expandierten Zustand zu dem radial komprimierten Zustand radial komprimiert wird;
Platzieren des Rahmens (12) in einem teilweise radial komprimierten Zustand zwischen dem radial expandierten Zustand und dem radial komprimierten Zustand; und
Koppeln der Segel (24) an den Rahmen (12), während sich die Segel (24) in einem relaxierten Zustand befinden und der Rahmen (12) sich in dem teilweise radial komprimierten Zustand befindet, so dass, wenn der Rahmen (12) zu dem radial expandierten Zustand expandiert wird, die Segel (24) in einer axialen Richtung Lose aufweisen, und wobei die Segel (24), wenn der Rahmen (12) zu dem radial komprimierten Zustand komprimiert wird, in der axialen Richtung gestreckt werden.

12. Verfahren nach Anspruch 11, wobei jedes Segel (24) einen Kuspenrandabschnitt (30), zwei gegenüberliegende Kommissurlaschen (28) und zwei gegenüberliegende Halsregionen (33) umfasst, wobei jede Halsregion (33) sich von einer der Kommissurlaschen (28) zu einem benachbarten Ende des Kuspenrandabschnitts (30) erstreckt, wobei jede Kommissurlasche (28) mit einer benachbarten Kommissurlasche (28) eines benachbarten Segels (24) gepaart wird, um eine Kommissur (42) zu bilden, und wobei Koppeln der Segel (24) an den Rahmen (12) Koppeln des Kuspenrandabschnitts (30) von jedem Segel (24) an den Rahmen (12) und Koppeln von jeder Kommissur (42) an den Rahmen (12) umfasst, so dass die Halsregionen (33) an dem Rahmen (12) unbefestigt bleiben.

13. Verfahren nach Anspruch 12, wobei der Kuspenrandabschnitt (30) von jedem Segel (24) mit Nähten (40) an eine Schürze (204) genäht wird, die mit Streben (18) des Rahmens (12) verbunden ist, wobei jede Kommissur (42) mit einem Kommissurträgerabschnitt des Rahmens (12) verbunden ist und jede Halsregion (33) eine sich axial erstreckende Lücke zwischen einer benachbarten Kommissur (42) und einem benachbarten Ende des Kuspenrandabschnitts (30) eines entsprechenden Segels (24) definiert.

14. Verfahren nach Anspruch 13, wobei der Kuspenrandabschnitt (30) jedes Segels (24) an:
a) eine diskrete Schürze; oder
b) dieselbe Schürze, die sich um einen gesamten Umfang einer inneren Oberfläche des Rahmens (12) erstreckt, genäht ist.

15. Verfahren nach Anspruch 13 oder Anspruch 14, wobei der Kuspenrandabschnitt (30) jedes Segels (24) an einen Verstärkungsstreifen (224) gegenüber der Schürze (204) genäht ist.

## Revendications

1. Valvule cardiaque prothétique (10) comprenant :
un bâti annulaire (12) qui est radialement compressible et expansible entre un état radialement comprimé et un état radialement expansé, dans lequel le bâti (12) s'allonge dans une direction axiale lorsqu'il est radialement comprimé de l'état radialement expansé à l'état radialement comprimé ; et
une pluralité de feuillets (24) disposés à l'intérieur du bâti (12) et configurés pour permettre au sang de s'écouler à travers le bâti (12) dans une première direction et bloquer l'écoulement du sang dans une seconde direction, opposée à la première direction ;
dans laquelle les feuillets (24) sont accouplés au bâti (12) de telle sorte que, lorsque le bâti est dans l'état radialement expansé, les feuillets présentent un mou (114) dans une direction axiale, dans laquelle, lorsque le bâti (12) est radialement comprimé à un état partiellement radialement comprimé entre l'état radialement expansé et l'état radialement comprimé, les feuillets (24) sont dans un état relâché, et dans laquelle, lorsque le bâti (12) est dans l'état radialement comprimé, les feuillets (24) sont étirés dans la direction axiale.

2. Valvule cardiaque prothétique selon la revendication 1, dans laquelle le bâti (12) comprend une pluralité d'entretoises (18) reliées de manière pivotante les unes aux autres au niveau d'une pluralité de joints de pivotement, dans laquelle les entretoises (18) peuvent pivoter les unes par rapport aux autres lorsque le bâti est radialement comprimé et dilaté.

3. Valvule cardiaque prothétique selon une quelconque revendication précédente, dans laquelle chaque feuillet (24) comprend une partie de bord de cuspide (30) reliée au bâti (12) et deux languettes de commissure opposées (28), dans laquelle chaque languette de commissure (28) est appariée à une languette de commissure adjacente d'un feuillet adjacent (24) pour former une commissure (42), qui est reliée au bâti (12).

4. Valvule cardiaque prothétique selon la revendication 3, dans laquelle chaque feuillet (24) comprend deux régions de col opposées (33), chacune s'étendant depuis l'une des languettes de commissure (28) jusqu'à une extrémité adjacente de la partie de bord de cuspide (30), dans laquelle les régions de col (33) ne sont pas attachées au bâti (12) de telle sorte que les régions de col (33) puissent s'étirer dans la direction axiale lorsque le bâti (12) est dans l'état radialement comprimé.

5. Valvule cardiaque prothétique selon la revendication 4, dans laquelle les régions de col (33) définissent des encoches (34) dans les feuillets (24).

6. Valvule cardiaque prothétique selon l'une quelconque des revendications 3 à 5, comprenant en outre une pluralité d'actionneurs (50) reliés au bâti (12), dans laquelle les actionneurs (50) sont configurés pour produire une expansion et une compression radiales du bâti (12).

7. Valvule cardiaque prothétique selon la revendication 6, dans laquelle chaque commissure (42) est reliée à l'un des actionneurs (50).

8. Valvule cardiaque prothétique selon l'une quelconque des revendications 3 à 7, comprenant en outre une pluralité de jupes en tissu (204), chacune étant suturée à la partie de bord de cuspide (30) d'un feuillet correspondant (24) et au bâti (12).

9. Valvule cardiaque prothétique selon la revendication 8, dans laquelle chaque jupe en tissu (204) présente des extrémités opposées qui sont alignées sur des extrémités adjacentes de la partie de bord de cuspide (30) d'un feuillet correspondant (24).

10. Valvule cardiaque prothétique selon l'une quelconque des revendications 8 à 9, comprenant en outre une pluralité de bandes de renforcement en tissu (224), chacune étant suturée à la partie de bord de cuspide (30) d'un feuillet correspondant (24) sur un côté opposé de la partie de bord de cuspide (30) à partir de la jupe en tissu (204) du même feuillet (24).

11. Procédé d'assemblage d'une valvule cardiaque prothétique (10), le procédé comprenant :
le placement d'une pluralité de feuillets (24) dans un bâti annulaire (12) qui est radialement compressible et expansible entre un état radialement comprimé et un état radialement expansé, dans lequel le bâti (12) s'allonge dans une direction axiale lorsqu'il est radialement comprimé de l'état radialement expansé à l'état radialement comprimé ;
le placement du bâti (12) dans un état partiellement radialement comprimé entre l'état radialement expansé et l'état radialement comprimé ; et
l'accouplement des feuillets (24) au bâti (12) pendant que les feuillets (24) sont dans un état relâché et que le bâti (12) est dans l'état partiellement radialement comprimé de telle sorte que, lorsque le bâti (12) est expansé vers l'état radialement expansé, les feuillets (24) présentent un mou dans une direction axiale, et dans lequel, lorsque le bâti (12) est comprimé à l'état radialement comprimé, les feuillets (24) sont étirés dans la direction axiale.

12. Procédé selon la revendication 11, dans lequel chaque feuillet (24) comprend une partie de bord de cuspide (30), deux languettes de commissure opposées (28), et deux régions de col opposées (33), chaque région de col (33) s'étendant de l'une des languettes de commissure (28) à une extrémité adjacente de la partie de bord de cuspide (30), dans lequel chaque languette de commissure (28) est appariée à une languette de commissure adjacente (28) d'un feuillet adjacent (24) pour former une commissure (42), et dans lequel l'accouplement des feuillets (24) au bâti (12) comprend l'accouplement de la partie de bord de cuspide (30) de chaque feuillet (24) au bâti (12) et l'accouplement de chaque commissure (42) au bâti (12) de telle sorte que les régions de col (33) restent non attachées au bâti (12).

13. Procédé selon la revendication 12, dans lequel la partie de bord de cuspide (30) de chaque feuillet (24) est suturée à une jupe (204), qui est reliée à des entretoises (18) du bâti (12) avec des sutures (40), chaque commissure (42) est reliée à une partie de support de commissure du bâti (12), et chaque région de col (33) définit un espace s'étendant axialement entre une commissure adjacente (42) et une extrémité adjacente de la partie de bord de cuspide (30) d'un feuillet correspondant (24).

14. Procédé selon la revendication 13, dans lequel la partie de bord de cuspide (30) de chaque feuillet est suturée à :
a) une jupe discrète ; ou
b) la même jupe qui s'étend sur toute la circonférence d'une surface interne du bâti (12).

15. Procédé selon la revendication 13 ou la revendication 14, dans lequel la partie de bord de cuspide (30) de chaque feuillet (24) est suturée à une bande de renforcement (224) en regard de la jupe (204).
